# EUROPÄISCHE PATENTSCHRIFT

(11) **EP 0 641 773 B1**
(45) Veröffentlichungstag und Bekanntmachung des Hinweises auf die Patenterteilung: **03.11.1999**
(21) Anmeldenummer: 94113365.4
(22) Anmeldetag: 26.08.1994
(51) Int. Cl.: C07C 303/22, C07C 303/32, C07C 309/40

(54) **Verfahren zur Herstellung von 4,4'-Dinitrostilben-2,2'-disulfonsäure**
Process for the preparation of 4,4'-dinitrostilbene-2,2'-disulfonic acid
Procédé pour la préparation d'acide 4,4'-dinitrostilbène-2,2'-disulfonique

(30) Priorität: 08.09.1993 DE 4330377
(43) Veröffentlichungstag der Anmeldung: 08.03.1995
(73) Patentinhaber: BAYER AG, 51368 Leverkusen (DE)
(72) Erfinder: Schnatterer, Albert, Dr., D-51373 Leverkusen (DE); Fiege, Helmut, Dr., D-51373 Leverkusen (DE)

(56) Entgegenhaltungen:
- EP-A- 0 026 154
- EP-A- 0 332 137

## Beschreibung

Die vorliegende Erfindung betrifft ein neues Verfahren zur Herstellung von 4,4'-Dinitrostilben-2,2'-disulfonsäure und deren Salzen.

4,4'-Dinitrostilben-2,2'-disulfonsäure ist ein wichtiges Zwischenprodukt zur Herstellung von optischen Aufhellern. Die Verbindung wird für diesen Einsatz in großen Mengen jährlich benötigt.

Verfahren zur industriellen Herstellung von 4,4'-Dinitrostilben-2,2'-disulfonsäure (DNS) sind seit langem bekannt. Bereits Ende des letzten Jahrhunderts wurden Methoden zur oxidativen Kondensation von 2 Mol 4-Nitrotoluol-2-sulfonsäure (p-NTSA) in wäßrigem Alkali entwickelt. Dabei wurden als Oxidationsmittel Sauerstoff (Luft) in Gegenwart eines Katalysators und Hypochlorit oder Chlor verwendet (vgl. z.B. Ber. dt. Chem. Ges. 30, 2097 - 3101 (1897); 31 1079 (1898); DRP 113 514). Mit diesen Verfahren konnten jedoch selbst unter Einsatz moderner Technik nur unbefriedigende Ausbeuten von 60 bis 75 % Dinitrostilbendisulfonsäure und deren Salzen erzielt werden (vgl. z.B. DE-A 2 258 530).

Neuere Varianten dieses Verfahrens der oxidativen Kondensation von 4-Nitrotoluol-2-sulfonsäure geben dem Sauerstoff als Oxidationsmittel den Vorzug gegenüber Hypochlorit. Diese Entwicklung verfolgt konsequent den Trend zur Kostenreduzierung, denn Sauerstoff in Form von Luft ist das billigste Oxidationsmittel.

Da bei der Sauerstoffoxidation ein Feststoff (Salze der 4-Nitrotoluol-2-sulfonsäure) mit einem Gas (O₂, Luft) in Gegenwart von Alkali und gegebenenfalls in Gegenwart eines Katalysators umgesetzt wird, ist ein wirksames Lösungsmittel verfahrensentscheidend.

Wasser bietet sich als Lösungsmittel im besonderen an. Zum einen sind die Reaktionsteilnehmer stark polare Komponenten, zum anderen sind sie in Form wäßriger Lösungen oder wasserfeuchter Feststoffe besonders preisgünstig verfügbar. Beispielsweise fallt die 4-Nitro-toluol-2-sulfonsäure bei der Herstellung durch Sulfonierung von 4-Nitrotoluol (vgl. z.B. EP-A 0 083 555) als wäßrige Lösung oder als wasserfeuchter Feststoff an. Die 4-Nitrotoluol-2-sulfonsäure kann in diesem Fall ohne Lösungsmittelwechsel und ohne weitere verfahrenstechnische Operation direkt in den Prozeß der DNS-Herstellung eingebracht werden. Auch die Handhabung von Alkali und gegebenenfalls einzusetzenden Metallsalzkatalysatoren erfolgt besonders wirtschaftlich in Form wäßriger Lösungen. Nachteilig am Verfahren der Oxidation in Wasser ist jedoch die schlechte Löslichkeit der 4-Nitrotoluol-2-sulfonsäure in wäßriger Natron- oder Kalilauge. Außerdem muß zum Erreichen einer hohen Selektivität vielfach bei niedrigen Konzentrationen und niedriger Reaktionsgeschwindigkeit gearbeitet werden, was letzlich eine unbefriedigende Raum-Zeit-Ausbeute mit sich bringt.

Die Luftoxidation in wäßriger Natronlauge wird beispielsweise in einer besonderen Verfahrensvariante in DD 240 200 beschrieben. In einem 2-stufigen Prozeß in Gegenwart katalytischer Mengen eines Mangansalzes wird in der 1. Stufe bei hoher p-NTS- und Alkalikonzentration unter teilweiser Ausfällung von 4,4'-Dinitrobibenzyl-2,2'-disulfonat und in der 2. Stufe bei niedriger Alkalikonzentration und hoher Verdünnung oxidiert. Die Ausbeuten an DNS werden mit 82 bis 85 % der Theorie angegeben, wobei das Beispiel mit der höchsten Ausbeute in der 2. Reaktionsstufe mit einer sehr niederen Konzentration arbeitet, die 57 g p-Nitrotoluolsulfonsäure pro 1000 cm³ Reaktionsvolumen entspricht. Den Beispielen kann außerdem eine Reaktionszeit für den Gesamtprozeß von 8 bis 9 Stunden entnommen werden.

Gemäß DE-A 3 409 171 kann die Konzentration an Dinitrostilbendisulfonsäure im Reaktionsgemisch auf über 30 Gew.-% erhöht und gleichzeitig die Reaktionszeit verkürzt werden, wenn die Luftoxidation in Gegenwart von Li⁺-Ionen, die überstöchiometrisch in Form von Lithiumhydroxid eingesetzt werden, durchgeführt wird. In den aufgeführten Beispielen erreichen die Ausbeuten je nach Arbeitsweise bis zu 86,5 % der Theorie an Dinitrostilbendisulfonsäure. Nachteilig bei diesem Verfahren ist der zusätzliche Schritt zur Abtrennung des Lithiums als Lithiumcarbonat im Anschluß an die Oxidation, wobei die Wiedergewinnung des Lithiumcarbonats nur 75-80 % beträgt. Um das Lithium wieder in den Prozeß zurückführen zu können muß das Lithiumcarbonat außerdem noch in das Lithiumhydroxid überführt werden. Die Wirkung des Lithiums beruht letztlich auf der verbesserten Löslichkeit des Lithiumsalzes der p-Nitrotoluolsulfonsäure in wäßrigem Alkali.

In der DE-A 35 195 52 ist ein Verfahren zur Herstellung von Dinitrostilbendisulfonsäure in wäßriger Suspension beschrieben, wobei K⁺-, Ca²⁺- oder Mg²⁺-Ionen während der Reaktion in dem Maße zugegeben werden wie die Dinitrostilbendisulfonsäure gebildet wird. Durch diese Maßnahme wird die Dinitrostilbendisulfonsäure als schwerlösliches Salz gefällt. Die Ausbeute an Dinitrostilbendisulfonsäure ist zwar hoch - in einem Beispiel, das in kontinuierlicher Fahrweise arbeitet, wird sie mit 94 % d.Th. angegeben - doch muß wieder in hoher Verdünnung von 5 - 10 % Dinitrostilbendisulfonsäure gearbeitet werden, wodurch letztlich wieder eine schlechte Raum-Zeit-Ausbeute resultiert. Außerdem muß den Beispielen zufolge ein Teil des Alkalis in Form des im Vergleich zum Natriumhydroxid deutlich teureren Kaliumhydroxids eingesetzt werden.

Außer Wasser werden als Lösungsmittel auch Ammoniak und dipolare aprotische Lösungsmittel beschrieben.

Gemäß EP-A 0 305 648 lassen sich unter Verwendung von flüssigem, wasserfreiem Ammoniak, Alkylderivaten des Ammoniaks und Gemischen dieser mit Wasser als Lösungsmittel sehr hohe Ausbeuten an Dinitrostilbendisulfonsäure erreichen, die in den Beispielen je nach Arbeitsweise mit bis zu 97 % angegeben werden. Nachteilig an diesem Verfahren ist die Verwendung eines Lösungsmittels, daß bei Raumtemperatur und unter den Reaktionsbedingungen gasförmig ist und zur Verflüssigung unter Druck gehandhabt werden muß. Dadurch ergibt sich ein erhöhter verfahrenstechnischer Aufwand, beispielsweise durch den notwendigen Einsatz von Druckapparaten. Ein weiterer Nachteil des Verfahrens resultiert aus dem Anfall der Reaktionswärme auf sehr niedrigem Temperaturniveau bedingt durch die tiefen Reaktionstemperaturen von 5 - 15°C. Die für Oxidationsreaktionen charakteristische hohe Reaktionswärme muß hier durch einen energie-intensiven Prozeß, beispielsweise durch Solekühlung, abgefangen werden.

Ebenfalls sehr hohe Ausbeuten an Dinitrostilbendisulfonsäure von bis zu 98 % lassen sich nach EP-A 0 332 137 durch Arbeiten in DMSO als Lösungsmittel oder nach EP-A 0 026 154 durch Arbeiten in dipolar aprotischen Lösungsmittel vom Säureamid-Typ, insbesondere DMF als Lösungsmittel, erzielen. In beiden Fällen werden dem Lösungsmittel niedere Alkohole als Co-Solvens zugesetzt, wobei im DMF-Verfahren Wasser weitestgehend ausgeschlossen werden muß während im DMSO-Verfahren geringe Wassermengen tolerierbar sind - doch beträgt die zugesetzte Wassermenge in den Beispielen mit den höchsten Ausbeuten weniger als 0,5 Gew.-% bezogen auf den Gesamteinsatz. Das wasserfreie Arbeiten stellt an die Lösungsmittel sowie an die Edukte beispielsweise die Nitrotoluolsulfonsäure, das Alkali und die Oxidationsluft hohe Qualitätsanforderungen. So müssen z.B. die Lösungsmittel vor dem Einsatz weitgehend wasserfrei gemacht werden. Ein entscheidender Nachteil der wasserfreien Verfahren betrifft den Einsatz der Nitrotoluolsulfonsäure. Die Nitrotoluolsulfonsäure, die bei der Herstellung nach den gängigen Verfahren der Sulfonierung von Nitrotoluol (vgl. z.B. EP-A 0 083 555) als wäßrige Lösung oder als wasserfeuchter Feststoff anfällt, kann nicht direkt in wasserfeuchter Form weiterverarbeitet werden. Vielmehr muß sie in einem der Oxidation vorgeschalteten Prozeß mit Alkali in das gewünschte Salz überführt und anschließend getrocknet werden. Nach EP-A 0 332 137 beispielsweise erfolgt dieser Prozeß durch Neutralisation mit wäßriger Natronlauge. Das Na-Salz der Nitrotoluolsulfonsäure fällt als Feststoff aus, wird von der wäßrigen Mutterlauge abgetrennt, in DMSO aufgenommen und durch Destillation getrocknet. Aufgrund dieser teuren zusätzlichen Maßnahmen ist trotz der hohen Ausbeute kein entscheidender wirtschaftlicher Vorteil gegenüber der Arbeitsweise in Wasser erkennbar.

Es bestand deshalb Bedarf an einem Verfahren, welches die Vorteile der nicht wäßrigen Lösungsmittel - hohe Raum-Zeit-Ausbeute und hohe chemische Ausbeute - mit den Vorteilen des Wasserverfahrens - kein Lösungsmittelwechsel nach der Nitrotoluolsulfonsäureproduktion, keine Nonwendigkeit zum Einsatz wasserfreier Edukte - verbindet, ohne daß mit einem Lösungsmittel wie Ammoniak, das unter den Reaktionsbedingungen gasförmig ist, gearbeitet werden muß.

Die vorliegende Erfindung betrifft ein Verfahren zur Herstellung von 4,4'-Dinitrostilben-2,2'-disulfonsäure und deren Salzen der Formel 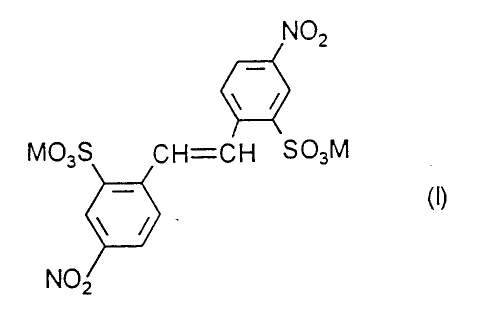 worin
- M: für Wasserstoff oder ein Alkalimetallion steht,
durch Behandeln von 4-Nitrotoluol-2-sulfonsäure mit Sauerstoff oder einem Sauerstoff enthaltenden Gas, bei einer Temperatur von 20 bis 80°C,
in Gegenwart von starken Basen, dadurch gekennzeichnet, daß die Oxidation in einem Gemisch aus Wasser und einem organischen Lösungsmittel durch geführt wird, wobei als
organisches Lösungsmittel wenigstens ein Lösungsmittel ausgewählt aus der Gruppe a) der geradkettigen oder verzweigten Alkohole mit 1 bis 4 C-Atomen und 1 oder 2 OH-Gruppen und/oder
b) der Dialkylether mit jeweils 1 bis 4 C-Atomen im geradkettigen oder verzweigten Alkylteil, die gegebenenfalls durch eine oder mehrere OH- oder NH₂-Gruppen substituiert sind, die z.B. Methyl-tert.-butylether, 2-Methoxyethanol oder 2-Methoxyethylamin;
der cyclischen Ether mit 3 bis 5 C-Atomen, die gesättigt oder olefinisch ungesättigt sein können wie z.B. Dioxan oder Furan;
oder der Polyether der Formel 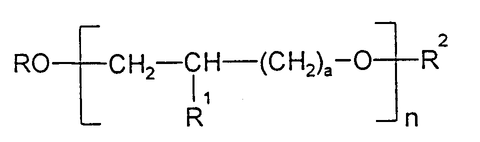 worin
- R, R¹ und R²: unabhängig voneinander für geradkettiges oder verzweigtes C₁-C₄-Alkyl stehen,
- a: für 0 bis 2 steht, und
- n: für 1 bis 8 steht,
oder Polyether der Reihe Ethylenglykoldimethylether, Diethylenglykoldimethylether, Triethylenglykoldimethylether oder deren Gemische bzw. Gemische dieser Polyether mit Methanol oder 1,2-Ethandiol eingesetzt wird und daß der Wasseranteil im Lösungsmittel 15 bis 90 Gew.-%, bezogen auf das Gemisch beträgt.

Das Alkalimetallion in der Definition von M ist bevorzugt Lithium, Natrium oder Kalium.

Beispielhaft seien hier genannt für die Alkohole a) das Methanol, Ethanol, 1-Propanol, 2-Propanol, 1-Butanol, 2-Butanol, tert.-Butanol, für die Diole das 1,2-Ethandiol, 1,2-Propandiol, 1,3-Propandiol, 1,2-Butandiol, 1,4-Butandiol. Für die unter b) genannten Verbindungen seien beispielhaft genannt für die Gruppe der Polyether die Methyl- und Ethylether des 1,2-Ethandiols, 1,2-Propandiols, 1,3-Propandiols, 1,2-Butandiols, 1,4-Butandiols; für die Gruppe der Etheralkohole das 2-Methoxyethanol, 2-Ethoxyethanol, Diethylenglykol, Triethylenglykol, Propylenglykol-1-methylether, Propylenglykol-1-ethylether, Dipropylenglykol; für die Gruppe der Etheramine das 2-Methoxyethylamin, 3-Methoxypropylamin, 3-Ethoxypropylamin, 2-Ethoxyethylamin.

Die organischen Lösungsmittel a) und b) können einzeln oder als Lösungsmittelgemisch von beliebiger Zusammensetzung eingesetzt werden. Bevorzugt ist die Verwendung von Gemischen von einem oder mehreren unter b) genannten Lösungsmittel mit mindestens einem unter a) genannten aliphatischen Alkohol und/oder Diol.

Besonders bevorzugt handelt es sich bei dem organischen Lösungsmittels um ein Gemisch aus einem oder mehreren Vertretern aus der unter b) genannten Gruppe der Polyether, Etheralkohole und Etheramine und einem oder mehreren Vertretern aus der Gruppe der unter a) genannten C₁-C₄-Alkohole und/oder C₁-C₄-Diole. Das Mengenverhältnis im organischen Lösungsmittelanteil zwischen dem Polyether, Etheralkohol und Etheramin einerseits und dem Alkohol und/oder Diol andererseits ist keiner strikten Begrenzung unterworfen und normalerweise so bemessen, daß eine ausreichende aber nicht notwendigerweise vollständige Löslichkeit des wäßrigen- und organischen Lösungsmittelanteils im erfindungsgemäßen Verfahren gewährleistet ist. Der Alkohol- und/oder Diolanteil im organischen Lösungsmittel kann zwischen 5 und 90 % bevorzugt zwischen 10 und 80 % variieren.

Besonders bevorzugt werden als organische Lösungsmittel Ethylenglykoldimethylether und/oder Diethylenglykoldimethylether und/oder Triethylenglykoldimethylether im Gemisch mit Methanol und/oder 1,2-Ethandiol eingesetzt.

Der Wasseranteil im erfindungsgemäß einzusetztenden Lösungsmittelgemisch aus Wasser und organischen Lösungsmittel kann über einen weiten Bereich variieren und liegt im allgemeinen zwischen 15 und 90 Gew.-%, besonders bevorzugt zwischen 20 und 80 Gew.-%, jeweils bezogen auf die Summe aus wäßrigem und organischem Lösungsmittelanteil.

Die 4-Nitrotoluol-2-sulfonsäure wird bevorzugt als wäßrige Lösung oder als Lösung in einem Gemisch aus Wasser und den erfindungsgemäß einzusetzenden organischen Lösungsmitteln in das Reaktionsgemisch eingebracht. Bevorzugt ist außerdem der Eintrag der 4-Nitrotoluol-2-sulfonsäure als wasserfeuchter Feststoff. Selbstverständlich kann die 4-Nitrotoluol-2-sulfonsäure auch in Form der Metallsalze, welche bei der Neutralisation mit den weiter unten beschriebenen starken Basen gebildet werden, eingesetzt werden, wenngleich diese Variante einen zusätzlichen Verfahrensschritt beinhalten kann.

Überraschenderweise lassen sich durch den Einsatz der erfindungsgemäßen Lösungsmittelgemische Ergebnisse bei der Kondensation von 4-Nitrotoluol-2-sulfonsäure erzielen, die den bisherigen Stand mit Wasser als Lösungsmittel deutlich übertreffen ohne daß der Nachteil des Ammoniaks oder des wasserfreien Verfahrens in Kauf genommen werden muß. Die Reaktionsgeschwindigkeit ist beispielsweise gegenüber dem Verfahren in Wasser deutlich erhöht; daraus ergibt sich nicht nur eine höhere Raum-Zeit-Ausbeute, auch die Reaktionstemperatur läßt sich auf ein Niveau absenken, das bisher für das Verfahren in Wasser nicht beschrieben ist. Die niedrigere Reaktionstemperatur ermöglicht wiederum eine selektivere Reaktion mit Ausbeuten über 90 % Dinitrostilbensulfonsäure. Ein weiterer Vorteil des erfindungsgemäßen Verfahrens ist die hohe Dinitrostilbensulfonsäurekonzentration im Reaktionsgemisch. Die weiter unten aufgeführten Beispiele zeigen, daß noch Reaktionsgemische mit 18 Gew.-% Dinitrostilbensulfonsäure hervorragende Ergebnisse bringen. Die unerwartet guten Ergebnisse sind umso überraschender als die bisherigen Verfahren in organischen Lösungsmitteln (vgl. EP 0 332 137 und EP 0 026 154) bevorzugt wasserfrei oder in Gegenwart von lediglich katalytischen Mengen Wasser arbeiten.

Als starke Basen kommen Verbindungen der Alkali- und Erdalkalimetalle in Frage, beispielsweise Metallhydroxide, Metallalkoholate und Metallamide und, soweit es sich um Alkoholate und Amide handelt, von Aluminium. Als wichtige Alkali- und Erdalkalimetalle seien beispielsweise Natrium, Kalium, Lithium, Calcium, Magnesium genannt. Als Alkoholate seien beispielsweise das Methylat, Ethylat, Isopropylat und das Propylenglykolat aufgeführt. Unter den genannten Basen sind die Hydroxide bevorzugt; insbesondere bevorzugt sind Natriumhydroxid und Kaliumhydroxid und Natriummethylat. Die genannten Basen können einzeln oder in beliebigem Gemisch untereinander eingesetzt werden.

Die einzusetzende Basenmenge kann sich in weiten Grenzen bewegen. Die Basenmenge hängt u.a. davon ab, ob man die p-Nitrotoluolsulfonsäure in der Säureform oder als Salz einsetzt. Da beim Einsatz der p-Nitrotoluolsulfonsäure in der Säureform 1 Äquivalent Base für die Neutralisation der Sulfonsäuregruppe verbraucht wird, wird die Base in wenigstens äquivalenten Mengen benötigt. Bevorzugt wird die Base in Mengen von 1,5 bis 8, vorzugsweise 2 bis 5 Basenäquivalenten eingesetzt.

Die Ausführung des erfindungsgemäßen Verfahrens unter Zusatz eines Katalysators kann vorteilhaft sein, ist aber nicht zwingend notwendig. Als Katalysatoren kommen insbesondere Verbindungen der Übergangsmetalle, beispielsweise von Co, Mn, Cr, Fe, Ni, Cu, V, Nb, Ta, Ru zum Einsatz. Mögliche Einsatzformen dieser Metalle sind deren Salze anorganischer Säuren, beispielsweise die Metallfluoride, -chloride, -sulfate, -nitrate, -carbonate, -phosphate; die Metalloxide und Metallhydroxide; die Metallsalze organischer Säuren, beispielsweise die Metallacetate, -oxalate, -phenolate, -benzoate, -salicylate; Komplexe dieser Metalle beispielsweise mit Acetylaceton, N,N'-Disalicyliden-ethylendiamin, Ethylendiamin, Diethylentriamin, Triethylentetramin, Tetraphenylporphin und Phthalocyanin.

Von besonderem Interesse sind unter den Metallkatalysatoren die Verbindungen des Mangans und Vanadiums in einer Menge von 0,0001 - 0,05 Moläquivalenten, bevorzugt 0,005 - 0,01 Moläquivalenten bezogen auf die 4-Nitro-toluol-2-sulfonsäure eingesetzt werden.

Als Oxidationsmittel für das erfindungsgemäße Verfahren kann reiner Sauerstoff oder Sauerstoff in verdünnter Form, beispielsweise in Form von Sauerstoff enthaltenden Gasen, eingesetzt werden. Die wirtschaftlich günstigste Form des erfindungsgemäß einzusetzenden Oxidationsmittels ist die atmosphärische Luft. Der Druck des Sauerstoffs bzw. des Sauerstoff enthaltenden Gases ist keiner besonderen Beschränkung unterworfen und kann zwischen 0,5 und 20 bar liegen, vorzugsweise bei 0,8 bis 10 bar, besonders bevorzugt in der Nähe des atmosphärischen Druckes. Der Sauerstoffgehalt ist bei Verwendung von Sauerstoff enthaltenden Gasen ebenfalls keiner Beschränkung unterworfen. Der Gehalt und die Menge an Sauerstoff richtet sich vor allem nach der Umsetzungsgeschwindigkeit. Es ist von Vorteil, den Sauerstoff oder die Luft im Reaktionsgemisch fein zu verteilen, beispielsweise unter Verwendung von Düsen oder Fritten.

Der Sauerstoff oder die Luft kann jedoch auch unter Verwendung geeigneter Rührer durch kräftiges Rühren in das Reaktionsgemisch eingezogen werden. Die Begasungsintensität, d.h. die pro Zeiteinheit zur Verfügung gestellte Gasmenge kann ebenfalls stark variiert werden und richtet sich beispielsweise nach der Reaktionsfreudigkeit der p-Nitrotoluol-2-sulfonsäure in dem jeweils verwendeten Reaktionsmedium aus dem erfindungsgemäßen Lösungsmittelgemisch, der Base und gegebenenfalls dem Katalysator. Die für den Einzelfall günstigsten Begasungsbedingungen, beispielsweise bezüglich des O₂-Gehaltes des Oxidationsgases und des Gasdruckes, lassen sich durch einfache Vorversuche ermitteln.

Die Reaktionstemperatur für das erfindungsgemäße Verfahren kann zwischen 0 und 100°C variieren. Bevorzugt wird jedoch bei einer Temperatur zwischen 20 und 80°C gearbeitet.

Das beim erfindungsgemäßen Verfahren anfallende Reaktionsgemisch kann direkt in die Prozesse eingespeist werden, die die Dinitrostilbendisulfonsäure als Ausgangsprodukt benötigen, beispielsweise in den Prozeß der Reduktion zu 4,4'-Diaminostilben-2,2'-disulfonsäure, welches ein wichtiges Ausgangsprodukt zur Herstellung optischer Aufheller ist. Die Dinitrostilbendisulfonsäure bzw. deren Salze können zu diesem Zweck aber auch zwischenisoliert werden. Die Isolierung erfolgt nach bekannten Verfahrensoperationen und ist im Einzelfall vom verwendeten organischen Lösungsmittel abhängig. Sind die Salze der Dinitrostilbendisulfonsäure beispielsweise in dem Reaktionsgemisch schwerlöslich, so können diese unmittelbar als Feststoff abgetrennt werden. Die verbleibende Mutterlauge kann gegebenenfalls mit 2-Nitrotoluolsulfonsäure und Base beaufschlagt und in den Oxidationsschritt des erfindungsgemäßen Verfahrens recyclisiert werden.

Ebenfalls möglich ist eine Abtrennung des organischen Lösungsmittelanteils aus dem Reaktionsgemisch unter Ausbildung einer wäßrigen Lösung oder Suspension der Salze der Dinitrostilbendisulfonsäure gegebenenfalls nach Neutralisaton überschüssiger Base mit Säure und gegebenenfalls nach Hinzufügen einer zusätzlichen Menge Wasser. Derartige wäßrige Lösungen oder Suspensionen können direkt in den oben genannten Folgeprozessen weiterverarbeitet werden. Sie können aber auch zur Kristallisation der Salze der Dinitrostilbendisulfonsäure herangezogen werden. Die Trennung von wäßrigen und organischen Lösungsmittelanteilen geschieht beispielsweise durch Destillation oder Phasentrennung oder durch eine Kombination dieser Verfahrensoperationen. Von Fall zu Fall kann es vorteilhaft sein durch Zusatz weiterer, bevorzugt unpolarer Lösungsmittel die Phasentrennung zu verbessern.

### Beispiel 1

In einem 2 l-Glasreaktor wurde ein Gemisch aus 400 ml Wasser, 210 g Methanol, 200 g Ethylenglykoldimethylether, 0,5 g Mangan(II)sulfat und 90 g Natriumhydroxid bei 40°C vorgelegt. Unter intensivem Begasen der Lösung mit 40 l Luft/h unter Normaldruck wurden 1/4 von insgesamt 510 g einer 30,5 %igen Lösung von 4-Nitrotoluol-2-sulfonsäure in einem Lösungsmittelgemisch aus 72 Gew.-% Wasser und 28 Gew.-% Ethylenglykoldimethylether während 10 min. zudosiert. Anschließend erhöhte man die Temperatur während 15 min. auf 50°C und dosierte den Rest der 4-Nitrotoluol-2-sulfonsäure-Lösung während 1 h simultan mit 110 g einer 27,3 %igen Natronlauge zu. Nach beendeter Dosierung ließ man noch insgesamt 5,5 h bei 50°C nachreagieren, wobei der zur Begasung eingeleitete Luftstrom 30 min. nach Ende der Dosierung auf 20 l/h und nach weiteren 2 h auf 10 l/h zurückgenommen wurde. Am Reaktionsende wurde die überschüßige Base mit 80 %iger Schwefelsäure neutralisiert. Die Ausbeute an 4,4'-Dinitrostilben-2,2'-dinatriumdisulfonat betrug nach HPLC-Analyse 90,4 % d.Th.

### Beispiel 2

Die Durchführung erfolgte wie in Beispiel 1 angegeben, mit dem Unterschied, daß Ethylenglykoldimethylether durch Diethylenglykoldimethylether ersetzt wurde. Die Reaktionsdauer nach vollständiger Dosierung der 4-Nitrotoluol-2-sulfonsäure betrug 6 Stunden.

Die Ausbeute an 4,4'-Dinitrostilben-2,2'-dinatriumdisulfonat nach HPLC-Analyse betrug 90, 1 % d.Th.

### Beispiel 3

Die Durchführung erfolgte wie in Beispiel 1 angegeben, mit dem Unterschied, daß Ethylenglykoldimethylether durch Triethylenglykoldimethylether ersetzt wurde. Die Reaktionsdauer nach vollständiger Dosierung der 4-Nitrotoluol-2-sulfonsäure betrug 6 Stunden.

Die Ausbeute an 4,4'-Dinitrostilben-2,2'-dinatriumdisulfonat betrug nach HPLC-Analyse 88,1 % d.Th.

### Beispiel 4

In einem 2 l-Glasreaktor wurde ein Gemisch aus 140 ml Wasser, 290 g Methanol, 250 g Ethylenglykoldimethylether, 0,3 g VOSO₄ · 5H₂O und 107 g Natriumhydroxid bei 40°C vorgelegt. Unter intensivem Begasen der Lösung mit einem Gasgemisch aus 60 l Luft und 30 l Stickstoff unter Normaldruck wurden 1/4 von ingesamt 780 g einer 32,9 %igen Lösung von 4-Nitrotoluol-2-sulfonsäure in einem Lösungsmittelgemisch aus 33 Gew.-% Wasser, 44 Gew.-% Ethylenglykoldimethylether und 22 Gew.-% Methanol während ca. 10 min. zudosiert. Anschließend wurde die Temperatur während 15 min auf 50°C gesteigert und der Rest der 4-Nitrotoluol-2-sulfonsäure-Lösung während 1,5 h simultan mit 161 g einer 37,9 %igen Natronlauge zudosiert. Nach beendeter Dosierung ließ man noch insgesamt 3,5 h bei 50°C nachreagieren. Während der gesamten Reaktion wurde der eingeleitete Luftstrom so nachreguliert, daß sich der O₂-Gehalt im Abgas, das den Reaktor verließ, zwischen 5 und 7 Vol.-% einpendelte. Am Reaktionsende wurde die überschüssige Base mit 80 %iger Schwefelsäure neutralisiert. Die Ausbeute an 4,4'-Dinitrostilben-2,2'-dinatriumdisulfonat betrug nach HPLC-Analyse 93,2 % der Theorie.

### Beispiel 5

Die Durchführung erfolgte wie in Beispiel 2 angegeben, mit dem Unterschied, daß ohne Zusatz von VOSO₄ gearbeitet wurde.

Die Ausbeute an 4,4'-Dinitrostilben-2,2'-dinatriumdisulfonat betrug nach HPLC-Analyse 88,1 % d.Th.

### Beispiel 6

Dieses Beispiel arbeitet mit einer p-Nitrotoluol-2-sulfonsäurekonzentration von 18 Gew.-% bezogen auf den Gesamtansatz.

In einem 2 l-Glasreaktor wurde ein Gemisch aus 140 ml Wasser, 290 g Methanol, 250 g Ethylenglykoldimethylether, 0,3 g VOSO₄ · 5H₂O und 107 g Natriumhydroxid bei 40°C vorgelegt. Unter intensivem Begasen der Lösung mit einem Gasgemisch aus 30 l Luft und 30 l Stickstoff unter Normaldruck wurden 1/6 von insgesamt 890 g einer 38,4 %igen Lösung von 4-Nitrotoluol-2-sulfonsäure in einem Lösungsmittelgemisch aus 45 Gew.-% Wasser, 36 Gew.-% Ethylenglykoldimethylether und 18 Gew.-% Methanol während ca. 10 min zudosiert. Anschließend wurde die Temperatur während 15 min. auf 55°C erhöht und der Rest der 4-Nitrotoluol-2-sulfonsäure-Lösung während 3 h simultan mit 186 g einer 46,2 %igen Natronlauge zudosiert. Nach beendeter Dosierung ließ man noch insgesamt 3 h bei 55°C nachreagieren. Während der gesamten Reaktion wurde der Oxidationsgasstrom durch Variation der Luft- und Stickstoffanteile so nachreguliert, daß der O₂-Gehalt im Abgas, das den Reaktor verließ, zwischen ca. 5 und 7 Vol-% lag. Am Reaktionsende wurde die überschüssige Base mit 80 %iger Schwefelsäure neutralisiert. Die Ausbeute an 4,4'-Dinitrostilben-2,2'-dinatriumdisulfonat betrug nach HPLC-Analyse 92,7 % d.Th.

### Beispiel 7

Die Reaktion wurde analog Beispiel 6 mit dem Unterschied durchgeführt, daß anstelle von VOSO₄ · 5H₂O als Katalysator 0,3 g MnSO₄ · H₂O eingesetzt wurden. Die Ausbeute an Dinatrium-4,4'-dinitrostilben-2,2-disulfonat betrug nach der HPLC-Analyse 93,9 % d. Th.

### Beispiel 8

In einem 2l-Glasreaktor wurde ein Gemisch aus 400 ml Wasser, 250 g 1,2-Ethandiol, 260 g Ethylenglykoldimethylether, 0,3 g MnSO₄ und 90 g Natriumhydroxid bei 40°C vorgelegt. Unter intensivem Begasen der Lösung mit 40 l Luft/h unter Normaldruck wurden 1/4 von insgesamt 510 g einer 32,1 %igen Lösung von 4-Nitrotoluol-2-sulfonsäure in einem Lösungsmittelgemsich aus 71 Gew.-% Wasser und 29 Gew.-% Ethylenglykoldimethylether während ca. 10 min. zudosiert. Anschließend wurde die Temperatur während 15 min. auf 55°C erhöht und der Rest der 4-Nitrotoluol-2-sulfonsäure-Lösung während 1h simultan mit 80 g einer 37,5 %igen Natronlauge zudosiert. Nach beendeter Dosierung rührte man noch 5h bei 55°C unter Reduzierung des Luftstroms auf 20 l/h nach. Am Reaktionsende lag eine dunkel gefärbte Lösung vor. Die überschüssige Base wurde mit 80 %iger Schwefelsäure neutralisiert, aus dem Reaktionsgemisch durch Zusatz einer ausreichenden Menge Wasser eine homogene Lösung hergestellt und aus dieser Lösung die Ausbeute durch HPLC-Analyse zu 84,9 % d.Th. bestimmt.

## Patentansprüche

1. Verfahren zur Herstellung von 4,4'-Dinitrostilben-2,2'-disulfonsäure und deren Salzen der Formel 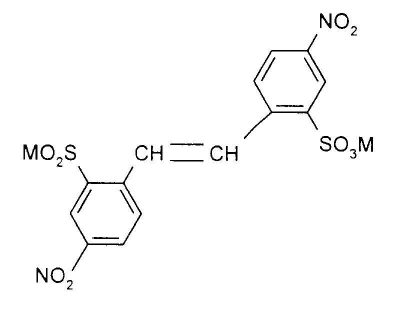 worin
M für Wasserstoff oder ein Alkalimetallion steht,
durch Behandeln von 4-Nitrotoluol-2-sulfonsäure mit Sauerstoff oder einem Sauerstoff enthaltenden Gas, bei einer Temperatur von 20 bis 80°C,
in Gegenwart von starken Basen, dadurch gekennzeichnet, daß die Oxidation in einem Gemisch aus Wasser und einem organischen Lösungsmittel durchgeführt wird, wobei als organisches Lösungsmittel wenigstens ein Lösungsmittel ausgewählt aus der Gruppe a) der geradkettigen oder verzweigten Alkohole mit 1 bis 4 C-Atomen und 1 oder 2 OH-Gruppen und/oder b) der Dialkylether mit jeweils 1 bis 4 C-Atomen im geradkettigen oder verzweigten Alkylteil, die gegebenenfalls durch eine oder mehrere OH- oder NH₂-Gruppen substituiert sind, der cyclischen Ether mit 3 bis 5 C-Atomen, die gesättigt oder olefinisch ungesättigt sein können oder der Polyether der Formel 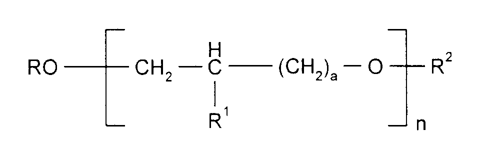 worin
R, R¹ und R² unabhängig voneinander für geradkettiges oder verzweigtes C₁-C₄-Alkyl stehen,
a für 0 bis 2 steht, und
n für 1 bis 8 steht,
oder Polyether der Reihe Ethylenglykoldimethylether, Diethylenglykoldimethylether, Triethylenglykoldimethylether oder deren Gemische bzw. Gemische dieser Polyether mit Methanol oder 1,2-Ethandiol eingesetzt wird und daß der Wasseranteil im Lösungsmittel 15 bis 90 Gew.-%, bezogen auf das Gemisch beträgt.

2. Verfahren gemäß Anspruch 1, dadurch gekennzeichnet, daß als Oxidationsmittel Luft eingesetzt wird.

3. Verfahren gemäß Ansprüchen 1 bis 2, dadurch gekennzeichnet, daß das Lösungsmittelgemisch aus Wasser und organischen Lösungsmitteln mindestens einen C₁-C₄-Alkohol und/oder C₁-C₄-Diol enthält.

4. Verfahren gemäß Ansprüchen 1 bis 3, dadurch gekennzeichnet, daß organische Lösungsmittel ausgewählt aus der Reihe Ethylenglykoldimethylether, Diethylenglykoldimethylether, Triethylenglykoldimethylether oder deren Gemische eingesetzt werden.

5. Verfahren gemäß Ansprüchen 1 bis 4, dadurch gekennzeichnet, daß als organisches Lösungsmittel Ethylenglykoldimethylether und/oder Diethylenglykoldimethylether und/oder Triethylenglykoldimethylether im Gemisch mit Methanol und/oder 1,2-Ethandiol zum Einsatz kommt.

6. Verfahren gemäß Ansprüchen 1 bis 5, dadurch gekennzeichnet, daß als starke Basen einer oder mehrere Vertreter aus der Gruppe der Hydroxide, Alkoholate und Amide von Alkali- und Erdalkalimetallen und Alkoholate und Amide von Aluminium in einer Menge von 1,5 - 8 Basenäquivalenten, bevorzugt in 2 - 5 Basenäquivalenten pro mol 4-Nitrotoluol-2-sulfonsäure eingesetzt werden.

7. Verfahren gemäß Ansprüchen 1 bis 6, dadurch gekennzeichnet, daß es in Gegenwart eines Katalysators aus der Gruppe der Übergangsmetallverbindungen, insbesondere Verbindungen des Co, Mn, Cr, Fe, Ni, Cu, V, Nb, Ta, Ru durchgeführt wird.

8. Verfahren nach Ansprüchen 1 bis 7, dadurch gekennzeichnet, daß als Katalysatoren Verbindungen des Mn und/oder V in einer Menge von 0,0001 - 0,05 Moläquivalenten, bevorzugt 0,005 - 0,01 Moläquivalenten bezogen auf die 4-Nitrotoluol-2-sulfonsäure eingesetzt werden.

## Claims

1. Process for preparing 4,4'-dinitrostilbene-2,2'-disulphonic acid and salts thereof of the formula 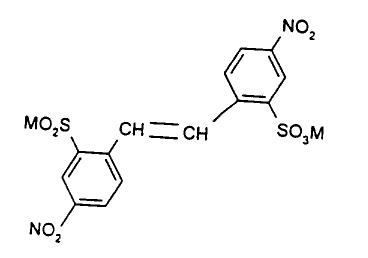 in which
M represents hydrogen or an alkali metal ion,
by treating 4-nitrotoluene-2-sulphonic acid with oxygen or an oxygen-containing gas at a temperature of from 20 to 80°C
in the presence of strong bases, characterized in that the oxidation is carried out in a mixture of water and an organic solvent, where the organic solvent used is at least one solvent selected from the group consisting of a) the straight-chain or branched alcohols having from 1 to 4 carbon atoms and 1 or 2 OH groups and/or b) the dialkyl ethers each having from 1 to 4 carbon atoms in the straight-chain or branched alkyl part, which are optionally substituted by one or more OH or NH₂ groups, the cyclic ethers having from 3 to 5 carbon atoms which can be saturated or olefinically unsaturated, or the polyethers of the formula 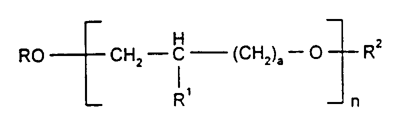 in which
R, R¹ and R², independently of one another, represent straight-chain or branched C₁-C₄-alkyl,
a represents a number from 0 to 2, and
n represents a number from 1 to 8,
or polyethers from the group consisting of ethylene glycol dimethyl ether, diethylene glycol dimethyl ether, triethylene glycol dimethyl ether or mixtures thereof or mixtures of these polyethers with methanol or 1,2-ethanediol, and in that the proportion of water in the solvent is from 15 to 90% by weight, based on the mixture.

2. Process according to Claim 1, characterized in that the oxidant used is air.

3. Process according to Claim 1 or 2, characterized in that the solvent mixture comprising water and organic solvents contains at least one C₁-C₄-alcohol and/or C₁-C₄-diol.

4. Process according to Claims 1 to 3, characterized in that the organic solvents used are selected from the group consisting of ethylene glycol dimethyl ether, diethylene glycol dimethyl ether, triethylene glycol dimethyl ether or mixtures thereof.

5. Process according to Claims 1 to 4, characterized in that the organic solvent used is ethylene glycol dimethyl ether and/or diethylene glycol dimethyl ether and/or triethylene glycol dimethyl ether in a mixture with methanol and/or 1,2-ethanediol.

6. Process according to Claims 1 to 5, characterized in that the strong bases used are one or more representatives selected from the group consisting of the hydroxides, alkoxides and amides of alkali and alkaline earth metals and alkoxides and amides of aluminium in an amount of 1.5-8 base equivalents, preferably 2-5 base equivalents, per mole of 4-nitrotoluene-2-sulphonic acid.

7. Process according to Claims 1 to 6, characterized in that it is carried out in the presence of a catalyst selected from the group consisting of transition metal compounds, in particular compounds of Co, Mn, Cr, Fe, Ni, Cu, V, Nb, Ta, Ru.

8. Process according to Claims 1 to 7, characterized in that the catalysts used are compounds of Mn and/or V in an amount of 0.0001-0.05 molar equivalents, preferably 0.005-0.01 molar equivalents, based on the 4-nitrotoluene-2-sulphonic acid.

## Revendications

1. Procédé pour la préparation d'acide 4,4'-dinitrostilbène-2,2'-disulfonique et de sels de celui-ci de la formule 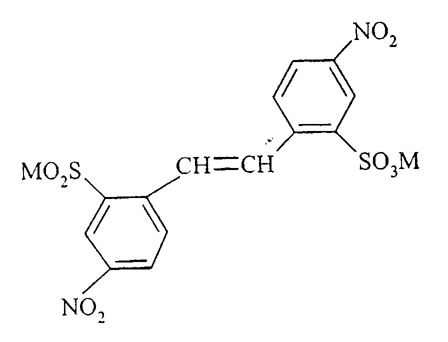 dans laquelle
M représente l'hydrogène ou un ion de métal alcalin,
par traitement d'acide 4-nitrotoluène-2-sulfonique avec de l'oxygène ou un gaz contenant de l'oxygène à une température de 20 à 80°C,
en présence de bases fortes, caractérisé en ce que l'on réalise l'oxydation dans un mélange constitué d'eau et d'un solvant organique,
au moins un solvant choisi parmi a) les alcools linéaires ou ramifiés avec de 1 à 4 atomes C et 1 ou 2 groupes OH et/ou b) les dialkyléthers avec à chaque fois de 1 à 4 atomes C dans une partie alkyle linéaire ou ramifiée, qui sont éventuellement substitués par un ou plusieurs groupes OH ou NH₂, les éthers cycliques avec de 3 à 5 atomes C qui peuvent être saturés ou oléfiniquement insaturés ou les polyéthers de la formule 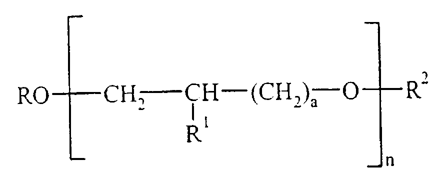
dans laquelle
R, R¹ et R² représentent indépendamment un groupe alkyle linéaire ou ramifié en C₁-C₄,
a est compris entre 0 et 2; et
n est compris entre 1 et 8,
ou les polyéthers de la série diméthyléther d'éthylèneglycol, diméthyléther de diéthylèneglycol, diméthyléther de triéthylèneglycol ou des mélanges de ceux-ci respectivement des mélanges de ces polyéthers avec du méthanol ou du 1,2-éthanediol, étant utilisé comme solvant organique et en ce que la part en eau dans le solvant est de 15 à 90% en poids, rapportés au mélange.

2. Procédé selon la revendication 1, caractérisé en ce que l'on utilise de l'air comme agent d'oxydation.

3. Procédé selon les revendications 1 à 2, caractérisé en ce que le mélange de solvants constitué d'eau et de solvants organiques contient au moins un alcool en C₁-C₄ et/ou un diol en C₁-C₄.

4. Procédé selon les revendications 1 à 3, caractérisé en ce que l'on utilise des solvants organiques choisis parmi la série diméthyléther d'éthylèneglycol, diméthyléther de diéthylèneglycol, diméthyléther de triéthylèneglycol ou des mélanges de ceux-ci.

5. Procédé selon les revendications 1 à 4, caractérisé en ce que l'on utilise comme solvant organique du diméthyléther d'éthylèneglycol et/ou du diméthyléther de diéthylèneglycol et/ou du diméthyléther de triéthylèneglycol en mélange avec du méthanol et/ou du 1,2-éthanediol.

6. Procédé selon les revendications 1 à 5, caractérisé en ce qu'on utilise comme bases fortes un ou plusieurs représentants parmi les hydroxydes, les alcoolates et les amides de métaux alcalins et alcalino-terreux et les alcoolates et les amides d'aluminium dans une quantité de 1,5-8 équivalents de base, de préférence de 2-5 équivalents de base par mole d'acide 4-nitrotoluène-2-sulfonique.

7. Procédé selon les revendications 1 à 6, caractérisé en ce qu'il est réalisé en présence d'un catalyseur du groupe des composés des métaux de transition, en particulier des composés de Co, Mn, Cr, Fe, Ni, Cu, V, Nb, Ta, Ru.

8. Procédé selon les revendications 1 à 7, caractérisé en ce que l'on utilise comme catalyseurs des composés du Mn et/ou du V dans une quantité de 0,0001-0,05 équivalents molaires, de préférence 0,005-0,01 équivalents molaires, rapportés à l'acide 4-nitrotoluène-2-sulfonique.
